# EUROPÄISCHE PATENTSCHRIFT

(11) **EP 0 853 953 B1**
(45) Veröffentlichungstag und Bekanntmachung des Hinweises auf die Patenterteilung: **09.04.2003**
(21) Anmeldenummer: 98100487.2
(22) Anmeldetag: 14.01.1998
(51) Int. Cl.: A61M 25/06, A61B 17/34

(54) **Punktionsset**
Puncturing set
Set de ponction

(30) Priorität: 17.01.1997 DE 19701546
(43) Veröffentlichungstag der Anmeldung: 22.07.1998
(73) Patentinhaber: Zumschlinge, Rolf, Dr.med., 82049 Pullach (DE)
(72) Erfinder: Zumschlinge, Rolf, Dr.med., 82049 Pullach (DE)
(74) Vertreter: Schlimme, Wolfram, Dr.-Ing.

(56) Entgegenhaltungen:
- DE-U- 8 603 357
- GB-A- 123 520
- US-A- 3 727 613
- US-A- 3 952 742
- US-A- 4 447 235
- US-A- 4 496 348
- US-A- 5 019 039
- US-A- 5 376 075

## Beschreibung

Die Erfindung betrifft ein Punktionsset gemäß dem Oberbegriff des Anspruchs 1, wie es allgemein in der Medizin verwendet wird, insbesondere zur Punktion von pathologischen mit Flüssigkeit gefüllten Körperhöhlen.

Ein derartiges Punktionsset ist aus der US 4,447,235 bekannt. Die US 4,447,235 offenbart ein braunülenartiges Punktionsinstrument, bei dem die Nadel nach dem Einbringen des Instruments aus der schlauchartigen Umhüllung herausgezogen wird. Nach dem Herausziehen der Nadel ist bei diesem Punktionsset eine Instabilität im schlauchartigen Ansatz gegeben.

Die US 5,376,075 A offenbart eine Anordnung aus Trokar und Katheter, gemäß dem Oberbegriff des ersten Anspruchs, zur Einführung in eine Blutbahn eines Patienten. Bei dieser Anordnung wird die Nadel durch eine Spiraldruckfeder in ihrer zurückgezogenen Position gehalten, wobei die Nadel jedoch problemlos gegen die Wirkung der Federkraft wieder nach vorne, also aus der Umhüllung heraus, verschoben werden kann.

Die DE 86 03 357 U betrifft einen Injektionsnadelkörper für flexible Injektionsnadeln. Derartige flexible Injektionsnadeln sind biegsam und können daher keine Versteifungsaufgabe während des Abziehens der Flüssigkeit wahrnehmen.

Aus der GB 123 520 A ist eine Infusionsnadel bekannt, bei der sich die Nadelspitze nach dem Einführen in den Körper in die rohrartige Umhüllung der Nadel durch axiales Verschieben der rohrartigen Umhüllung zurückziehen läßt. Die rohrartige Umhüllung wird dabei in ihrer Axialbewegung begrenzt von einer Schraube, deren Spitze in einer Längsnut gelegen ist, welche im Gehäuse vorgesehen ist. Durch die in der Axialnut geführte Schraubenspitze ist die Axialbewegung der Umhüllung in beide Richtungen begrenzt.

Die US 3,727,613 A offenbart eine Anordnung zum Setzen eines Katheters, bei dem eine Punktionsnadel innerhalb eines sie umgebenden Rohrs axial verfahrbar ist. Hierbei handelt es sich um einen Venenkatheter, bei dem die Nadelspitze durch axiales Vorwärtsschrauben der sie umgebenden Umhüllung in die Umhüllung zurückgefahren werden kann, um nach dem Entnehmen des Venenkatheters die Gefahr der Verletzung des Behandlungspersonals zu verhindern.

Die US 3,952,742 A offenbart eine Katheteranordnung für den Einsatz im Herzen mit entsprechenden Dichtungsvorrichtungen zum Abdichten der Außenwandung des die Nadel umgebenden Schlauchs an der Penetrationsstelle der Herzwand. Nach dem Einsetzen der Kathederanordnung wird die Nadel entfernt und die Anordnung dient beispielsweise zum Einbringen von Medikamenten in das Herz.

Die US 5,019,039 A offenbart eine Punktionsvorrichtung, bei der nach dem Setzen der Punktionsvorrichtung die Nadel ebenfalls aus dem schlauchartigen Ansatz herausgezogen wird, sodaß dieser während des Abziehens der Flüssigkeit instabil ist.

Die US 4,496,348 offenbart lediglich eine Arretierung für die Nadel einer Braunüle in ihrer aus der schlauchartigen Umhüllung hervorstehenden Position. Ein Schutz des Patienten ist mit dieser Vorrichtung nicht möglich.

Gattungsgemäße Punktionssets werden eingesetzt, um unerwünschte Flüssigkeitsansammlungen aus Körperhöhlen zu entfernen oder um zu diagnostischen Zwecken Proben einer Körperflüssigkeit zu entnehmen. Üblicherweise werden für derartige Punktionen Hohlnadeln, sogenannte Kanülen oder Trokare, eingesetzt, die durch die Körperwandung in den zu entleerenden Körperhohlraum eingestochen werden, woraufhin die Flüssigkeit durch die Hohlnadel abgezogen wird. Nachteilig ist bei derartigen bekannten Vorrichtungen, daß die während des Abziehens der Flüssigkeit im Körperhohlraum verbleibende Nadel mit ihrer Spitze das den Hohlraum begrenzende Körpergewebe verletzen kann.

Bekannt ist außerdem die Verwendung von sog. Braunülen zur Punktion. Eine Braunüle ist ein Einstichinstrument, das einen kanülenartigen Kunststoffschlauch aufweist, in dem eine Hohlnadel angeordnet ist, deren Spitze über das vordere Ende des Kunststoffschlauches hervorsteht. Eine Braunüle wird üblicherweise in den Körper, beispielsweise in eine Vene, eingeführt, indem mittels der Hohlnadel die Körperwandung durchstochen wird und indem anschließend der Kunststoffschlauch soweit wie erforderlich in den Körper eingeschoben wird. Danach wird die Nadel aus dem Kunststoffschlauch herausgezogen und entfernt, so daß der Kunststoffschlauch im Körper, beispielsweise in der Vene, verbleibt, um daran eine Infusion anzuschließen. Die Verwendung von Braunülen zur Punktion weist allerdings den Nachteil auf, daß der im Körperhohlraum verbleibende Teil des Kunststoffschlauches während des Abziehens der Körperflüssigkeit durch Bewegungen des Körpers oder von dessen Organen abknicken kann, wodurch ein weiterer Abtransport der Flüssigkeit nicht mehr oder nur erschwert möglich ist.

Insbesondere kritisch sind die beiden vorgenannten Punktionsverfahren bei der Punktion von Pleuroergüssen, d. h. von Flüssigkeitsansammlungen in dem die Lunge umgebenden Körperhohlraum. Hierbei ist einerseits zu vermeiden, die sich beim Atmen bewegende Lunge mittels der Nadelspitze zu verletzen und andererseits ist auch für einen längeren Zeitraum ein zuverlässiger Flüssigkeitsabtransport zu gewährleisten, um auch größere Flüssigkeitsansammlungen zuverlässig entfernen zu können.

Es ist daher die Aufgabe der vorliegenden Erfindung, ein Punktionsset zu schaffen, mit dem eine zuverlässige Entnahme von Flüssigkeit aus Körperhohlräumen möglich ist, ohne daß dabei Körpergewebe in unerwünschter Weise verletzt wird.

Diese Aufgabe wird durch das im Anspruch 1 angegebene Punktionsset gelöst.

Das erfindungsgemäße Punktionsset ist versehen mit einem Gehäuse, das einen Schlauchansatz aufweist, einer innerhalb des Gehäuses angeordneten rohrförmigen Punktionsnadel, die den Schlauchansatz axial im wesentlichen durchdringt und einer an der vom Schlauchansatz abgewandten Seite gelegenen Abflußvorrichtung, wobei die Punktionsnadel zwischen einer vorgeschobenen Position, in der ein vorderes, als Spitze ausgebildetes, zuflußseitiges Ende der Punktionsnadel in Axialrichtung aus dem Schlauchansatz an dessen freiem Ende hervorsteht, und einer zurückgezogenen Position, in der die Spitze innerhalb des Schlauchansatzes gelegen ist, axial verfahrbar ist, wobei die Abflußvorrichtung am rückwärtigen, abflußseitigen Ende der Punktionsnadel vorgesehen ist, wobei eine zwischen der Punktionsnadel und dem Gehäuse wirksame Dichtungsanordnung vorgesehen ist und wobei eine Arretiervorrichtung für die Punktionsnadel in ihrer zurückgezogenen Position vorgesehen ist.

Die axial verfahrbare Punktionsnadel erfüllt beim erfindungsgemäßen Punktionsset zwei Aufgaben, nämlich zum ersten die bekannte Aufgabe, beim Einführen des Punktionssets die Körperwandung zu durchstechen, und andererseits die Aufgabe, während des Abziehens der Flüssigkeit den Schlauchansatz zu stabilisieren und so ein Abknicken des Schlauchansatzes zu verhindern. Dabei ist die Nadel so weit in den Schlauchansatz zurückgezogen, daß ihre Spitze nicht mehr über das vordere Ende des Schlauchansatzes hervorsteht, so daß die Nadelspitze keine Verletzungsgefahr für Körpergewebe während des Abführens der Flüssigkeit darstellt. Damit während des Abführens der Flüssigkeit durch die hohle Punktionsnadel keine Luft von außen durch den zwischen der Außenseite der Punktionsnadel und der Innenseite des Schlauchansatzes gebildeten Ringkanal hindurchtreten kann und auf diese Weise das Abziehen der Flüssigkeit erschweren oder verhindern kann, ist zwischen dem Umfang der Punktionsnadel und dem Gehäuse eine Dichtungsanordnung vorgesehen. Durch die Arretiervorrichtung für die Punktionsnadel wird gewährleistet, daß die Punktionsnadel während des Abziehens der Flüssigkeit nicht ungewollt wieder in ihre vorgeschobene Position verfahren werden kann, wodurch sichergestellt ist, daß die Spitze der Punktionsnadel innerhalb des Schlauchansatzes verbleibt.

Vorzugsweise weist die Dichtungsanordnung eine Radialdichtung auf.

In einer bevorzugten Ausführungsform ist die Punktionsnadel über eine gewindeartige Drehverbindung, vorzugsweise mit steiler Gewindesteigung, mit dem Gehäuse gekoppelt.

Besonders vorteilhaft ist eine Weiterbildung, bei der die Dichtungsanordnung einen inneren, am Umfang der Punktionsnadel ausgebildeten Dichtungsabschnitt und einen äußeren, im Inneren des Gehäuses ausgebildeten Dichtungsabschnitt aufweist und wobei die beiden Dichtungsabschnitte in der vorgeschobenen Position der Punktionsnadel axial voneinander beabstandet sind. Bei dieser Ausführungsform wird die Abdichtung erst dann herbeigeführt, wenn die Punktionsnadel in ihre zurückgezogene Position verfahren wird.

Bei einer weiteren vorteilhaften Ausgestaltung ist der innere Dichtungsabschnitt von der schräg zur Nadelachse verlaufenden Mantelfläche eines den Umfang der Punktionsnadel kegelförmig umgebenden Dichtkörpers ausgebildet, dessen Kegelbasis der Spitze der Punktionsnadel zugewandt ist, wobei der äußere Dichtungsabschnitt an einer Innenkegelfläche im Gehäuse ausgebildet ist. Hierdurch wird eine besonders zuverlässige Abdichtung geschaffen, wobei bevorzugt zumindest eine der beiden Kegelflächen auch ballig konvex ausgebildet sein kann.

Vorzugsweise weist die Dichtungsanordnung einen inneren, am Umfang der Punktionsnadel ausgebildeten Dichtungsabschnitt und einen äußeren, im Inneren des Gehäuses ausgebildeten Dichtungsabschnitt auf, wobei die beiden Dichtungsabschnitte in der vorgeschobenen Position der Punktionsnadel axial voneinander beabstandet sind, wobei vorzugsweise der innere Dichtungsabschnitt von der schräg zur Nadelachse verlaufenden Mantelfläche eines den Umfang der Punktionsnadel kegelförmig umgebenden Dichtkörpers ausgebildet ist, dessen Kegelbasis der Spitze der Punktionsnadel zugewandt ist, und der äußere Dichtungsabschnitt an einer Innenkegelfläche im Gehäuse ausgebildet ist.

Alternativ weist die Dichtungsanordnung vorzugsweise einen inneren, am Umfang der Punktionsnadel ausgebildeten Dichtungsabschnitt und einen äußeren, im Inneren des Gehäuses ausgebildeten Dichtungsabschnitt auf, wobei die beiden Dichtungsabschnitte in der vorgeschobenen Position der Punktionsnadel axial voneinander beabstandet sind, wobei vorzugsweise der innere Dichtungsabschnitt von der Mantelfläche eines den Umfang der Punktionsnadel kugelförmig umgebenden Dichtkörpers gebildet ist, und wobei der äußere Dichtungsabschnitt an einer teilkugelförmigen Innenfläche eines Hohlraums im Gehäuse ausgebildet ist. Bei dieser Ausführungsform kann der kugelförmige Dichtkörper in den teilkugelförmig ausgebildeten Hohlraum des Gehäuses eintreten und dort eine zuverlässige Dichtwirkung herbeiführen.

Besonders vorteilhaft ist es, wenn die Arretiervorrichtung als kombinierte Dichtungs- und Rastanordnung ausgebildet ist, da hierdurch eine einfache und wirkungsvolle Arretierung der Punktionsnadel erzielbar ist.

Bei einer bevorzugten Ausführungsform ist das rückwärtige Ende der Punktionsnadel axialfest in einer jochartigen Kappe gelagert, die über ein Gewinde oder eine gewindeartige Drehverbindung mit dem Gehäuse verbunden ist. Hierbei kann die Nadel durch Verdrehen der Kappe relativ zum Gehäuse zuverlässig in ihre zurückgezogene Position verfahren werden, wobei Markierungen am Gehäuse und an der Kappe vorgesehen sein können, die die jeweilige Position der Punktionsnadel anzeigen.

Alternativ dazu kann die Punktionsnadel mit einem Außengewinde versehen sein, das mit einem im Gehäuse ausgebildeten Innengewinde im Schraubeingriff steht.

Sind gemäß einer weiteren Ausführungsform im Bereich des freien Endes des Schlauchansatzes Queröffnungen in der Schlauchwandung vorgesehen, so wird auch dann eine zuverlässige Ableitung der Flüssigkeit erzielt, wenn beispielsweise die stirnseitige Öffnung des Schlauchansatzes durch Gewebeteilchen oder andere Körper verstopft sein sollte.

Ist an der Außenseite des Schlauchansatzes über dessen Umfang ein radial ausdehnbarer Wandungsabschnitt vorgesehen, dessen Inneres über einen Druckkanal mit einem Druckanschluß im rückwärtigen Gehäuseabschnitt in Verbindung steht, so kann der ausdehnbare Wandungsabschnitt nach dem Einführen des vorderen Endes des Punktionssets in den Körperhohlraum von außen über den Druckanschluß und den Druckkanal aufgeblasen werden, um sich auszudehnen und so eine sichere Arretierung des Punktionssets im Körperhohlraum zu gewährleisten. Ein ungewolltes Herausrutschen des Punktionssets aus dem Körper wird damit verhindert. Zum Entfernen des Punktionssets muß lediglich das in den ausdehnbaren Wandungsabschnitt hineingedrückte Fluid wieder nach außen abgelassen werden, um den ausdehnbaren Wandungsabschnitt wieder auf einen Außendurchmesser zu bringen, der im wesentlichen dem Außendurchmesser des Schlauchansatzes entspricht.

Mündet das rückwärtige Ende der Punktionsnadel in eine Gehäusekammer, die in Verbindung mit der Abflußvorrichtung steht, so kann die Abflußvorrichtung unmittelbar am Gehäuse vorgesehen sein.

Weist die Abflußvorrichtung ein Drei-Wege-Ventil auf, dessen Eingang mit der Punktionsnadel in Fluidverbindung steht, dessen erster Ausgang zum Ansetzen einer Spritze ausgebildet ist und dessen zweiter Ausgang über einen Schlauch mit einem Sammelgefäß in Fluidverbindung steht, so kann mittels der angesetzten Spritze ein Absaugen von Fluid aus dem Körperhohlraum in die Spritze erfolgen, wobei das in die Spritze gesaugte Fluid nach dem Umschalten des Drei-Wege-Ventils aus der Spritze in das Sammelgefäß gedrückt werden kann. Hierdurch wird das Entleeren des Körperhohlraums beschleunigt, da die Spritze zum Absaugen der Flüssigkeit nicht ständig neu angesetzt werden muß.

Vorteilhaft ist auch, wenn im Gehäuse oder im rückwärtigen Bereich der Punktionsnadel ein Sichtfenster vorgesehen ist. Mittels eines derartigen Sichtfensters kann festgestellt werden, ob beim Einführen des Punktionssets in den Körperhohlraum der Ort mit dem zu entfernenden Flüssigkeitsvorrat getroffen worden ist, da beim Eindringen des Punktionssets in den Flüssigkeitsvorrat Flüssigkeit entweder durch die Punktionsnadel oder seitlich neben der Punktionsnadel entlang durch den Schlauchansatz in das Gehäuse strömt und schließlich im Sichtfenster sichtbar wird.

Die Erfindung wird nachfolgend anhand eines Beispiels unter Bezugnahme auf die Zeichnung näher erläutert; in dieser zeigt:
- **Fig. 1.**: ein erfindungsgemäßes Punktionsset in einer ersten Ausführungsform mit der Punktionsnadel in ihrer vorgeschobenen Position;
- **Fig. 2**.: das Punktionsset aus Fig. 1 mit in der zurückgezogenen Position befindlicher Punktionsnadel;
- **Fig. 3.**: eine alternative Ausführungsform mit einer abgewandelten Dichtung und einem abgewandelten Betätigungsmechanismus für die Punktionsnadel;
- **Fig. 4.**: eine weitere alternative Ausführungsform des Betätigungsmechanismus' für die Punktionsnadel;
- **Fig. 5.**: eine abgewandelte Ausgestaltung der Abdichtung der Punktionsnadel;
- **Fig. 6.**: noch eine andere Ausführungsform der Abdichtung der Punktionsnadel und
- **Fig. 7.**: eine Ausführungsform des erfindungsgemäßen Punktionssets mit einer Abwandlung im Bereich des vorderen Endes.

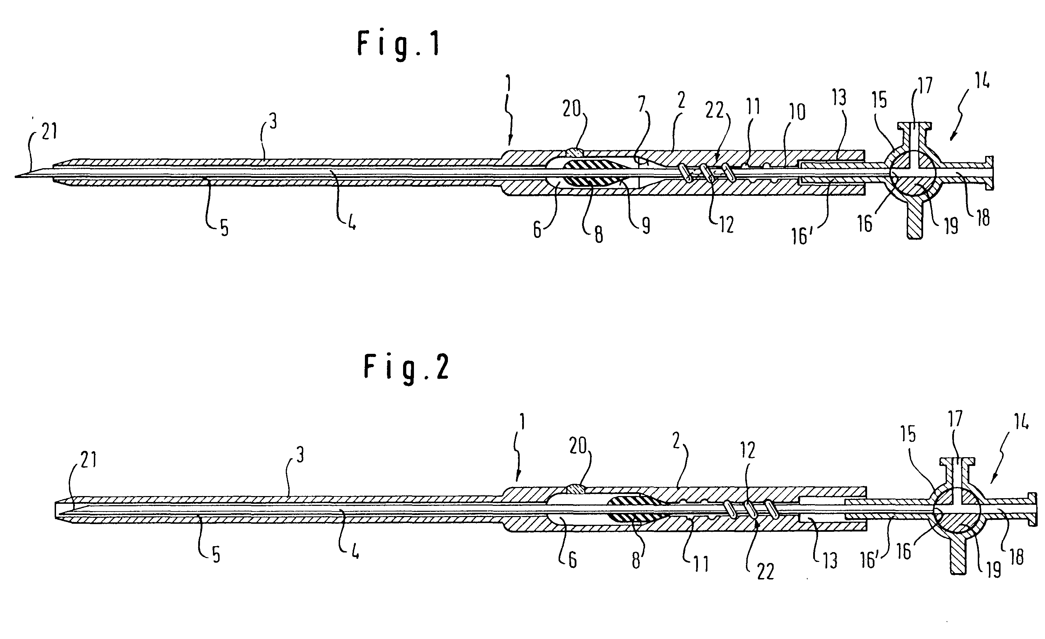

In Fig. 1 ist ein erfindungsgemäßes Punktionsset dargestellt, welches ein Gehäuse 1 aufweist, das aus einem rückwärtigen Gehäuseabschnitt größeren Durchmessers 2 und einem vorderen Schlauchansatz 3 besteht. Der rückwärtige Gehäuseabschnitt 2 und der vordere Schlauchansatz 3 sind koaxial zueinander ausgebildet und können entweder einstückig ausgebildet sein oder als Einzelteile, die mittels bekannter Verbindetechniken wie beispielsweise durch Kleben miteinander verbunden sind. Das Gehäuse 1 mit seinem rückwärtigen Gehäuseabschnitt 2 und dem Schlauchansatz 3 besteht vorzugsweise aus einem Kunststoffmaterial, das physiologisch unbedenklich ist.

Im Inneren des Gehäuses 1 ist eine Punktionsnadel 4 axial verfahrbar angeordnet. Die Punktionsnadel 4 durchdringt den Schlauchansatz 3 axial in voller Länge, wobei zwischen dem Außenumfang der Punktionsnadel 4 und dem Innenumfang des Schlauchansatzes 3 ein ringförmiger Kanal 5 ausgebildet ist.

Im Inneren des rückwärtigen Gehäuseabschnitts 2 mündet der Kanal 5 in eine vordere Gehäusekammer 6, die an ihrem rückwärtigen, vom Schlauchansatz 3 abgewandten Ende einen sich konusartig verjüngenden äußeren Dichtungsabschnitt 7 aufweist. Im Bereich der vorderen Gehäusekammer 6 ist die Punktionsnadel an ihrem Außenumfang von einem Dichtungskörper 8 umgeben, der an seinem rückwärtigen Ende einen ebenfalls konisch ausgebildeten inneren Dichtungsabschnitt 9 bildet, wobei der Spitzenwinkel des jeweiligen Konus' der beiden Dichtungsabschnitte im wesentlichen gleich ist. In der Darstellung in Fig. 1 sind der äußere Dichtungsabschnitt 7 und der innere Dichtungsabschnitt 9 axial voneinander entfernt.

Von der vorderen Gehäusekammer 6 erstreckt sich eine axiale Gehäusebohrung 10 nach hinten, also vom Schlauchansatz 3 weg. Die axiale Gehäusebohrung 10 ist an ihrem Umfang mit Gewindenuten 11 versehen, die eine steile Gewindesteigung aufweisen. Im Bereich der axialen Gehäusebohrung 10 ist die Punktionsnadel 4 an ihrem Außenumfang mit radial hervorstehenden Gewindegängen 12 versehen, die in die Gewindenuten 11 eingreifen und deren Steigung der Steigung der Gewindenuten 11 entspricht.

Im Bereich des rückwärtigen Endes des Gehäuses 1 ist der rückwärtige Gehäuseabschnitt 2 mit einer axialen Sackbohrung 13 versehen, in die die axiale Gehäusebohrung 10 zentral mündet. Das rückwärtige Ende der Punktionsnadel 4 erstreckt sich dabei aus der axialen Gehäusebohrung 10 heraus in die axiale Sackbohrung 13. Im Bereich der axialen Sackbohrung 13 ist das rückwärtige Ende der Punktionsnadel 4 mit einem das rückwärtige Ende der Punktionsnadel abdichtend übergreifenden Anschlußstück 16' einer Abflußvorrichtung 14 verbunden, von der in Fig. 1 nur ein Drei-Wege-Ventil 15 gezeigt ist.

Das Drei-Wege-Ventil 15 besitzt einen Eingang 16, in den das rückwärtige Ende der Punktionsnadel 4 abdichtend eingesetzt ist und welches mit der Punktionsnadel 4 drehfest verbunden ist. Weiterhin besitzt das Drei-Wege-Ventil 15 einen ersten Ausgang 17, der im rechten Winkel zur Axialrichtung des Gehäuses 1 steht, sowie einen zweiten Ausgang 18, der im wesentlichen koaxial zur Achse des Gehäuses 1 gelegen ist. Im Inneren des Drei-Wege-Ventils 15 ist ein Ventilkörper 19 in bekannter Weise vorgesehen, der zwischen einer ersten Stellung, in der der Eingang 16 mit dem ersten Ausgang 17 verbunden ist, und einer zweiten Stellung, in der der erste Ausgang 17 mit dem zweiten Ausgang 18 verbunden ist, umschaltbar ist. An den zweiten Ausgang 18 ist ein nicht gezeigtes Sammelgefäß über einen nicht gezeigten Schlauch in bekannter Weise angeschlossen. Der erste Ausgang 17 ist so ausgebildet, daß eine - nicht gezeigte - Spritze angesetzt werden kann.

Der rückwärtige Gehäuseabschnitt 2 ist zudem im Bereich der vorderen Gehäusekammer 6 mit einem Sichtfenster 20 versehen, welches einen Einblick von außen in die vordere Gehäusekammer 6 ermöglicht.

In Fig. 1 ist das Punktionsset in einer Position dargestellt, in der die Spitze 21 der Punktionsnadel 4 aus dem Schlauchansatz 3 an dessen vorderem freien Ende hervorsteht.

In Fig. 2 ist dasselbe Punktionsset wie in Fig. 1 dargestellt, allerdings in einer zurückgezogenen Position der Punktionsnadel 4, in der sich die Spitze 21 der Punktionsnadel 4 innerhalb des Schlauchansatzes 3 befindet. Um diese Position einnehmen zu können, muß die Punktionsnadel 4 durch Verdrehen des Drei-Wege-Ventils 15 aus der in Fig. 1 gezeigten vorgeschobenen Position in die in Fig. 2 gezeigte zurückgezogene Position verdreht werden, wobei die Drehbewegung vom Gewinde 22, das durch die Gewindenuten 11 und die Gewindegänge 12 gebildet wird, in eine Axialbewegung der Punktionsnadel 4 umgesetzt wird. Bei diesem Zurückschrauben der Punktionsnadel 4 wandert auch der Dichtungskörper 8 zusammen mit der Punktionsnadel 4 nach hinten, so daß sich der innere Dichtungsabschnitt 9 an den äußeren Dichtungsabschnitt 7 abdichtend anlegt.

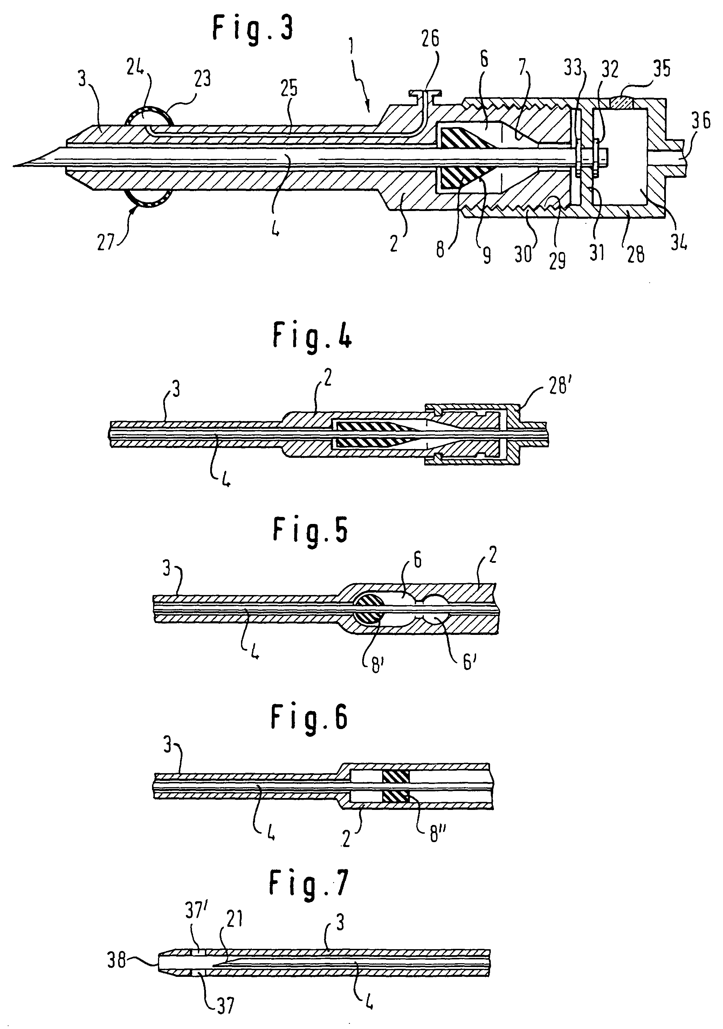

Fig. 3 ist eine im Durchmesser vergrößerte und in der Länge verkürzte Darstellung einer alternativen Ausführungsform des erfindungsgemäßen Punktionssets. In dem in Fig. 3 gezeigten Beispiel sowie in den in den folgenden Figuren gezeigten Beispielen betreffen dieselben Bezugszeichen wie in dem Beispiel nach den Fig. 1 und 2 auch dieselben Merkmale.

Im vorderen Bereich des Schlauchansatzes 3 ist an der Außenseite des Schlauchansatzes 3 über dessen Umfang ein radial ausdehnbarer Wandungsabschnitt 23 vorgesehen, der beispielsweise aus Gummi oder einem anderen flexiblen und elastischen Material gebildet sein kann. Der vom flexiblen Wandungsabschnitt 23 und dem Umfang des Schlauchansatzes 3 umschlossene Innenraum 24 ist über einen in der Wandung des Schlauchansatzes 3 ausgebildeten Druckkanal 25 mit einem Druckanschluß 26 im rückwärtigen Gehäuseabschnitt 2 verbunden. Über den Druckanschluß 26 und den Druckkanal 25 kann beispielsweise Druckluft oder ein Inertgas unter Druck in den Innenraum 24 eingeleitet werden, woraufhin sich der flexible Wandungsabschnitt 23 und der Innenraum 24 ausdehnen und einen ballonartigen Ringwulst um die Außenseite des Schlauchansatzes 3 herum bilden. Ein derartiger Ringwulst 27 kann verhindern, daß das Punktionsset ungewollt aus dem Körper herausrutscht.

Am rückwärtigen Ende ist das Punktionsset der Fig. 3 mit einer Kappe 28 versehen, die mit einem Innengewinde 29 auf ein Außengewinde 30 am hinteren Ende des rückwärtigen Gehäuseabschnitts 2 aufgeschraubt ist. Im Inneren der Kappe 28 ist eine Querwand 31 vorgesehen, die sich im wesentlichen rechtwinklig zur Längsachse der Punktionsnadel 4 erstreckt und in der das hintere Ende der Punktionsnadel 4 zumindest axialfest wie in einem Joch gelagert ist, was durch einen hinteren Federklemmring 32 angedeutet ist, der auf der Rückseite der Querwand 31 das rückwärtige Ende der Punktionsnadel 4 axial festklemmt, sowie durch einen vorderen Federklemmring 33, der auf der vorderen Seite der Querwand 31 die Punktionsnadel 4 umgreift und dort axial festlegt. Durch Verdrehen der Kappe 28 kann die Punktionsnadel 4 somit zwischen ihren beiden Extrempositionen verfahren werden.

Im rückwärtigen Bereich der Kappe 28 ist ein Hohlraum 34 ausgebildet, in den das rückwärtige Ende der Punktionsnadel 4 mündet. Im Bereich des Hohlraums 34 ist in der Wandung der Kappe 28 ein Sichtfenster 35 vorgesehen.

Auf der Rückseite der Kappe 28 ist ein Ausflußkanal 36 vorgesehen, der mit dem Hohlraum 34 in Fluidverbindung steht und der beispielsweise mit einem Drei-Wege-Ventil 15, das wie im Beispiel der Fig. 3 auch einstückig mit der Kappe 28 ausgebildet sein kann, verbunden ist.

In Fig. 4 ist schematisch eine Abwandlung des Punktionssets nach Fig. 3 dargestellt, wobei die Punktionsnadel 4, in der Kappe 28' ähnlich wie in der Querwand 31 der Kappe 28 des Beispiels in Fig. 3 jochartig in Axialrichtung fixiert ist. Die Kappe 28' ist jedoch nicht über ein Gewinde mit dem rückwärtigen Gehäuseabschnitt 2 verbunden, sondern über einen Rastmechanismus, bei dem die Kappe 28' durch axiales Verschieben (gemeinsam mit der Punktionsnadel 4) in zweiEndstellungen einrastbar ist, die beispielsweise durch entsprechende Rastgegenmittel am Außenumfang des rückwärtigen Gehäuseabschnitts 2 definiert sind, in welche Rastmittel der Kappe 28' eingreifen können. Alternativ zur Axialverschiebung der Kappe 28' kann der Rastmechanismus auch als Rast-Drehmechanismus gestaltet sein, wobei die an der Kappe 28' vorgesehenen Rastmittel entlang einer schraubenartigen Spur auf der Oberfläche des rückwärtigen Gehäuseabschnitts 2 verdreht werden können und wobei an den Enden der Spur die Rastgegenmittel vorgesehen sind, in die die Rastmittel zur Festlegung der Kappe 28' in den Endpositionen einrasten.

Eine andere Ausführungsform ist in Fig. 5 dargestellt. Dort ist der Dichtungskörper 8' als die Punktionsnadel 4 umgebende Kugel ausgebildet, die - wie auch der Dichtungskörper 8 - vorzugsweise aus einem elastischen Material gebildet ist. Die rückwärtige Innenumfangswand der vorderen Gehäusekammer 6 im rückwärtigen Gehäuseabschnitt 2 begrenzt einen Innendurchmesser, der geringfügig kleiner ist als der Außendurchmesser des kugelartigen Dichtungskörpers 8', so daß der Dichtungskörper 8' unter Verformung durch die von der rückwärtigen Wand der vorderen Gehäusekammer 6 gebildete Engstelle hindurchtreten kann und eine dahinter angeordnete hintere Gehäusekammer 6' erreichen kann, die ebenfalls kugelförmig oder zumindest teilkugelförmig ausgebildet ist und die den kugelförmig ausgebildeten Dichtungskörper 8' in der zurückgezogenen Position der Punktionsnadel 4 abdichtend aufnimmt.

Eine besonders einfache alternative Ausführungsform der Abdichtung ist in Fig. 6 dargestellt, wobei im rückwärtigen Gehäuseabschnitt 2 zwischen dem Innenumfang des rückwärtigen Gehäuseabschnitts 2 und dem Außenumfang der Punktionsnadel 4 eine Radialdichtung 8" in Form eines O-Rings oder einer Stopfbuchse in bekannter Weise ausgebildet ist.

In Fig. 7 ist das vordere Ende des Schlauchansatzes 3 einer abgewandelten Ausführungsform des erfindungsgemäßen Punktionssets dargestellt, bei der im Bereich des freien Endes des Schlauchansatzes 3 Queröffnungen 37, 37' in der Schlauchwandung vorgesehen sind. Die Nadelspitze 21 ist dabei in der zurückgezogenen Position der Punktionsnadel 4 hinter den Queröffnungen 37, 37' gelegen. Auf diese Weise kann bei einer Verstopfung der Öffnung 38 am freien Ende des Schlauchansatzes 3 die abzuleitende Flüssigkeit über die Queröffnungen 37, 37' in das Innere des Schlauchansatzes 3 und in das Innere der Punktionsnadel 4 eintreten.

Das Durchführen einer Punktion wird nachfolgend anhand der Fig. 1 und 2 erläutert. Zunächst wird die Punktionsnadel 4 in ihre in Fig. 1 dargestellte vorgeschobene Position gebracht, in der die Spitze 21 der Punktionsnadel 4 aus dem vorderen Ende des Schlauchansatzes 3 hervorsteht. Das Punktionsset wird dann durch die Körperwandung in den Körperhohlraum eingeführt, wobei die Spitze 21 der Punktionsnadel 4 und zum Teil auch der Schlauchansatz 3 in den Körper eindringen. Sobald im Sichtfenster 20 zu erkennen ist, daß Körperflüssigkeit in die vordere Gehäusekammer 6 eingedrungen ist, kann beurteilt werden, ob der gewünschte Zielort im Körperhohlraum erreicht ist. Danach wird die Punktionsnadel 4 in ihre in Fig. 2 dargestellte zurückgezogene Position verfahren, in der die Nadelspitze 21 vollständig innerhalb des Schlauchansatzes 3 aufgenommen ist. In diesem Zustand stabilisiert die Punktionsnadel 4 den aus Kunststoff bestehenden Schlauchansatz 3 und verhindert dessen Abknicken, wobei gleichzeitig gewährleistet ist, daß die Nadelspitze keine unerwünschten Verletzungen in der Wandung des Körperhohlraums bewirken kann. Ist das Punktionsset mit der in Fig. 3 dargestellten aufblasbaren balgartigen Ringwulst 27 versehen, so wird diese jetzt aufgeblasen, so daß sie sich beim geringfügigen Herausziehen des Punktionssets aus dem Körper an der Innenseite der Körperwandung anlegt und ein weiteres Herausrutschen des Punktionssets aus dem Körper verhindert. Die zu entfernende Körperflüssigkeit wird nun entweder aufgrund des ihr eigenen Drucks unmittelbar in ein Sammelgefäß geleitet, wobei die Körperflüssigkeit durch das Innere der Punktionsnadel 4 in die Abflußvorrichtung 14 geleitet wird, oder die Körperflüssigkeit wird in eine am ersten Ausgang 17 des Drei-Wege-Ventils 15 angeschlossene Spritze gesaugt und wird dann aus dieser nach dem Umschalten des Drei-Wege-Ventils 15 durch den zweiten Ausgang 18 in das Sammelgefäß entleert. Nachdem die gewünschte Menge an Flüssigkeit aus dem Körperhohlraum abgezogen worden ist, wird gegebenenfalls die Luft aus dem balgartigen Ringwulst 27 abgelassen und dann wird das Punktionsset aus dem Körper herausgezogen.

### Bezugszeichenliste

- 1: Gehäuse
- 2: rückwärtiger Gehäuseabschnitt
- 3: Schlauchansatz
- 4: Punktionsnadel
- 5: Kanal
- 6: vordere Gehäusekammer
- 6': hintere Gehäusekammer
- 7: äußerer Dichtungsabschnitt
- 8: Dichtungskörper
- 8': Dichtungskörper
- 8'': Dichtungskörper
- 9: innerer Dichtungsabschnitt
- 10: axiale Gehäusebohrung
- 11: Gewindenuten
- 12: Gewindegänge
- 13: axiale Sackbohrung
- 14: Abflußvorrichtung
- 15: Drei-Wege-Ventil
- 16: Eingang
- 16': Anschlußstück
- 17: erster Ausgang
- 18: zweiter Ausgang
- 19: Ventilkörper
- 20: Sichtfenster
- 21: Nadelspitze
- 22: Gewinde
- 23: flexibler Wandungsabschnitt
- 24: Innenraum
- 25: Druckkanal
- 26: Druckanschluß
- 27: Ringwulst
- 28: Kappe
- 28': Kappe
- 29: Innengewinde
- 30: Außengewinde
- 31: Querwand
- 32: hinterer Federklemmring
- 33: vorderer Federklemmring
- 34: Hohlraum
- 35: Sichtfenster
- 36: Ausflußkanal
- 37: Queröffnungen
- 37': Queröffnung
- 38: Öffnung

## Patentansprüche

1. Punktionsset mit
- einem Gehäuse (1), das einen Schlauchansatz (3) aufweist,
- einer innerhalb des Gehäuses (1) angeordneten rohrförmigen Punktionsnadel (4), die den Schlauchansatz (3) axial im wesentlichen durchdringt und
- einer an der vom Schlauchansatz abgewandten Seite gelegenen Abflußvorrichtung,
wobei
- die Punktionsnadel (4) zwischen einer vorgeschobenen Position, in der ein vorderes, als Spitze (21) ausgebildetes, zuflußseitiges Ende der Punktionsnadel (4) in Axialrichtung aus dem Schlauchansatz (3) an dessen freiem Ende hervorsteht, und einer zurückgezogenen Position, in der die Spitze (21) innerhalb des Schlauchansatzes (3) gelegen ist, axial verfahrbar ist, und wobei
- die Abflußvorrichtung am rückwärtigen, abflußseitigen Ende der Punktionsnadel (4) vorgesehen ist, **dadurch gekennzeichnet**
- **daß** eine zwischen der Punktionsnadel (4) und dem Gehäuse (1) wirksame Dichtungsanordnung vorgesehen ist und
- **daß** eine Arretiervorrichtung für die Punktionsnadel (4) in ihrer zurückgezogenen Position vorgesehen ist.

2. Punktionsset nach Anspruch 1,
**dadurch gekennzeichnet,**
- **daß** die Dichtungsanordnung eine Radialdichtung (8") aufweist.

3. Punktionsset nach einem der vorhergehenden Ansprüche,
**dadurch gekennzeichnet,**
- **daß** die Punktionsnadel über eine gewindeartige Drehverbindung, vorzugsweise mit steiler Gewindesteigung, mit dem Gehäuse (1) gekoppelt ist.

4. Punktionsset nach einem der vorhergehenden Ansprüche,
**dadurch gekennzeichnet,**
- **daß** die Dichtungsanordnung einen inneren, am Umfang der Punktionsnadel (4) ausgebildeten Dichtungsabschnitt (9) und einen äußeren, im Inneren des Gehäuses (1) ausgebildeten Dichtungsabschnitt (7) aufweist,
- **daß** die beiden Dichtungsabschnitte (7, 9) in der vorgeschobenen Position der Punktionsnadel (4) axial voneinander beabstandet sind, wobei vorzugsweise
- der innere Dichtungsabschnitt (9) von der schräg zur Nadelachse verlaufenden Mantelfläche eines den Umfang der Punktionsnadel (4) kegelförmig umgebenden Dichtkörpers (8) ausgebildet ist, dessen Kegelbasis der Spitze (21) der Punktionsnadel (4) zugewandt ist, und
- der äußere Dichtungsabschnitt (7) an einer Innenkegelfläche im Gehäuse (1) ausgebildet ist.

5. Punktionsset nach einem der vorhergehenden Ansprüche,
**dadurch gekennzeichnet,**
- **daß** die Dichtungsanordnung einen inneren, am Umfang der Punktionsnadel (4) ausgebildeten Dichtungsabschnitt (9) und einen äußeren, im Inneren des Gehäuses (1) ausgebildeten Dichtungsabschnitt (7) aufweist,
- **daß** die beiden Dichtungsabschnitte (7, 9) in der vorgeschobenen Position der Punktionsnadel (4) axial voneinander beabstandet sind, wobei vorzugsweise
- der innere Dichtungsabschnitt von der Mantelfläche eines den Umfang der Punktionsnadel (4) kugelförmig umgebenden Dichtkörpers (8') gebildet ist, und
- der äußere Dichtungsabschnitt an einer teilkugelförmigen Innenfläche eines Hohlraums (6') im Gehäuse (1) ausgebildet ist.

6. Punktionsset nach einem der vorhergehenden Ansprüche,
**dadurch gekennzeichnet,**
- **daß** die Arretiervorrichtung als kombinierte Dichtungs- und Rastanordnung ausgebildet ist.

7. Punktionsset nach einem der vorhergehenden Ansprüche,
**dadurch gekennzeichnet,**
- **daß** das rückwärtige Ende der Punktionsnadel (4) axialfest in einer jochartigen Kappe (28) gelagert ist, die über ein Gewinde (22; 29, 30) oder eine gewindeartige Drehverbindung, vorzugsweise mit steiler Gewindesteigerung, mit dem Gehäuse (1) gekoppelt ist.

8. Punktionsset nach einem der vorhergehenden Ansprüche,
**dadurch gekennzeichnet,**
- **daß** die Punktionsnadel (4) mit einem Außengewinde (12) versehen ist, das mit einem im Gehäuse (1) ausgebildeten Innengewinde (11) im Schraubeingriff steht.

9. Punktionsset nach einem der vorhergehenden Ansprüche,
**dadurch gekennzeichnet,**
- **daß** im Bereich des freien Endes des Schlauchansatzes (3) Queröffnungen (37, 37') in der Schlauchwandung vorgesehen sind.

10. Punktionsset nach einem der vorhergehenden Ansprüche,
**dadurch gekennzeichnet,**
- **daß** an der Außenseite des Schlauchansatzes (3) über dessen Umfang ein radial ausdehnbarer Wandungsabschnitt (23) vorgesehen ist, dessen Inneres (24) über einen Druckkanal (25) mit einem Druckanschluß (26) im rückwärtigen Gehäuseabschnitt (2) in Verbindung steht.

11. Punktionsset nach einem der vorhergehenden Ansprüche
**dadurch gekennzeichnet,**
- **daß** das rückwärtige Ende der Punktionsnadel (4) in eine Gehäusekammer (34) mündet, die in Verbindung mit der Abflußvorrichtung (14) steht.

12. Punktionsset nach einem der vorhergehenden Ansprüche,
**dadurch gekennzeichnet,**
- **daß** an die Abflußvorrichtung (14) ein Drei-Wege-Ventil (15) aufweist, dessen Eingang (16) mit der Punktionsnadel (4) in Fluidverbindung steht, dessen erster Ausgang (17) zum Ansetzen einer Spritze ausgebildet ist und dessen zweiter Ausgang (18) mit einem Sammelgefäß in Fluidverbindung steht.

13. Punktionsset nach einem der vorhergehenden Ansprüche,
**dadurch gekennzeichnet,**
- **daß** im Gehäuse (1) oder im rückwärtigen Bereich der Punktionsnadel (4) ein Sichtfenster (20; 35) vorgesehen ist.

## Claims

1. Puncturing set comprising
- a housing (1) with a hose extension (3),
- a tubular puncturing needle (4) disposed in housing (1) and extending axially substantially through the hose extension (3), and
- a fluid withdrawal mechanism arranged at the side facing away from the hose extension,
wherein
- the puncturing needle (4) is axially movable between an extended position, in which a forward upstream end of the puncturing needle (4), which is configured as pointed end (21), extends axially past a free end of the hose extension(3), and a retracted position, in which the pointed end (21) is disposed inside hose extension (3), and wherein
- the fluid withdrawal mechanism is arranged at the aft, downstream end of the puncturing needle (4),
**characterized in that**
- a sealing arrangement effective between the puncturing needle (4) and the housing (1) is provided, and
- a locking mechanism for locking the puncturing needle (4) in its retracted position is provided.

2. Puncturing set according to claim 1,
**characterized in that**
- the sealing arrangement comprises a radial seal (8").

3. Puncturing set according to any one of the preceding claims,
**characterized in that**
- the puncturing needle is coupled to the housing (1) by means of a thread-like screw connection, preferably having a steep thread pitch.

4. Puncturing set according to any one of the preceding claims,
**characterized in that**
- the sealing arrangement comprises an inner sealing section (9) which is disposed on the periphery of the puncturing needle (4), and an outer sealing section (7) formed in the interior of the housing (1),
- the two sealing sections (7, 9) are axially spaced apart from each other when the puncturing needle (4) is in its extended position, wherein preferably
- the inner sealing section (9) is defined by the surface area, that is angularly inclined relative to the axis of the needle, of a sealing body (8) conically surrounding the periphery of the puncturing needle (4), the conical base of which is facing the pointed end (21) of the puncturing needle (4), and
- the outer sealing portion (7) is disposed at an inwardly facing conical surface in the housing (1).

5. Puncturing set according to any one of the preceding claims,
**characterized in that**
- the sealing arrangement comprises an inner sealing section (9) which is disposed on the periphery of the puncturing needle (4), and an outer sealing section (7) formed in the interior of the housing (1),
- the two sealing sections (7, 9) are axially spaced apart from each other when the puncturing needle (4) is in its extended position, wherein preferably
- the inner sealing section is defined by the surface area of a sealing body (8') spherically surrounding the periphery of the puncturing needle (4), and
- the outer sealing section is disposed at a partially spherically shaped inner surface of a hollow space (6') in the housing (1).

6. Puncturing set according to any one of the preceding claims,
**characterized in that**
- the locking mechanism is formed as a combined sealing and latching mechanism.

7. Puncturing set according to any one of the preceding claims,
**characterized in that**
- the aft end of the puncturing needle (4) is axially immovably engaged in a yoke-like cap (28) connected to housing (1) via a thread (22; 29, 30) or a thread-like screw connection, preferably of steep thread pitch.

8. Puncturing set according to any one of the preceding claims,
**characterized in that**
- the puncturing needle (4) includes an outer thread (12) engaged with an inner thread (11) provided in the housing (1).

9. Puncturing set according to any one of the preceding claims,
**characterized in that**
- lateral openings (37, 37') are provided in the hose wall proximate a free end of the hose extension (3).

10. Puncturing set according to any one of the preceding claims,
**characterized in that**
- a radially expandable wall segment (23) is provided extending about a circumference and arranged exteriorly of the hose extension (3) the inner space (24) of the wall segment being connected to a pressure connection (26) at the aft housing segment (2) through a pressure channel (25).

11. Puncturing set according to any one of the preceding claims,
**characterized in that**
- the aft end of the puncturing needle (4) opens into a housing chamber (34) that is in connection with the fluid withdrawal mechanism (14).

12. Puncturing set according to any one of the preceding claims,
**characterized in that**
- the fluid withdrawal mechanism (14) comprises a three-way valve (15) having an inlet (16) in fluid communication with the puncturing needle (4), a first outlet (17) of which being adapted to be coupled to a syringe, and a second outlet (18) of which being in fluid connection with a collection container.

13. Puncturing set according to any one of the preceding claims,
**characterized in that**
- a sight window (20; 35) is arranged in either housing (1) or an aft portion of the puncturing needle (4).

## Revendications

1. Ensemble de ponction comportant
- un boîtier (1) qui comprend un talon tubulaire (3),
- une aiguille de ponction tubulaire (4) agencée à l'intérieur du boîtier (1) et traversant sensiblement axialement le talon tubulaire (3), et
- un dispositif d'évacuation situé sur le côté détourné du talon tubulaire, dans lequel
- l'aiguille de ponction (4) est axialement mobile entre une position avancée dans laquelle une extrémité antérieure côté afflux réalisée sous forme de pointe (21) de l'aiguille de ponction (4) fait saillie en direction axiale hors du talon tubulaire (3) à son extrémité libre, et une position retirée dans laquelle la pointe (21) est située à l'intérieur du talon tubulaire (3), et dans lequel
- le dispositif d'évacuation est prévu à l'extrémité postérieure côté évacuation de l'aiguille de ponction (4),
**caractérisé en ce que**
- il est prévu un organe d'étanchement agissant entre l'aiguille de ponction (4) et le boîtier (1), et
- il est prévu un dispositif d'arrêt pour l'aiguille de ponction (4) dans sa position retirée.

2. Ensemble de ponction selon la revendication 1, **caractérisé en ce que** l'organe d'étanchement comprend un joint radial (8").

3. Ensemble de ponction selon l'une des revendications précédentes, **caractérisé en ce que** l'aiguille de ponction est accouplée au boîtier (1) via une liaison rotative en forme de pas de vis, de préférence à pas raide.

4. Ensemble de ponction selon l'une des revendications précédentes, **caractérisé en ce que**
- l'organe d'étanchement comprend un tronçon d'étanchement intérieur (9) réalisé à la périphérie de l'aiguille de ponction (4) et un tronçon d'étanchement extérieur (7) réalisé à l'intérieur du boîtier (1),
- dans la position avancée de l'aiguille de ponction (4), les deux tronçons d'étanchement (7, 9) sont écartés axialement l'un de l'autre, dans lequel de préférence
- le tronçon d'étanchement intérieur (9) est réalisé par la surface enveloppe, s'étendant en oblique par rapport à l'axe de l'aiguille, d'un corps d'étanchement (8) entourant en forme conique la périphérie de l'aiguille de ponction (4), corps dont la base conique est tournée vers la pointe (21) de l'aiguille de ponction (4), et
- le tronçon d'étanchement extérieur (7) est réalisé sur une surface conique intérieure dans le boîtier (1).

5. Ensemble de ponction selon l'une des revendications précédentes, **caractérisé en ce que**
- l'organe d'étanchement comprend un tronçon d'étanchement intérieur (9) réalisé à la périphérie de l'aiguille de ponction (4) et un tronçon d'étanchement extérieur (7) réalisé à l'intérieur du boîtier (1),
- dans la position avancée de l'aiguille de ponction (4), les deux tronçons d'étanchement (7, 9) sont écartés axialement l'un de l'autre, dans lequel de préférence
- le tronçon d'étanchement intérieur (9) est réalisé par la surface enveloppe d'un corps d'étanchement (8) entourant en forme sphérique la périphérie de l'aiguille de ponction (4), et
- le tronçon d'étanchement extérieur est réalisé sur une surface intérieure partiellement sphérique d'une cavité (6') dans le boîtier (1).

6. Ensemble de ponction selon l'une des revendications précédentes, **caractérisé en ce que** le dispositif d'arrêt est réalisé sous forme d'organe d'étanchement et d'enclenchement combiné.

7. Ensemble de ponction selon l'une des revendications précédentes, **caractérisé en ce que** l'extrémité postérieure de l'aiguille de ponction (4) est montée de façon axialement fixe dans un capuchon (28) en forme d'étrier qui est accouplé au boîtier (1) via un pas de vis (22 ; 29, 30) ou via une liaison rotative en forme de pas de vis, de préférence à pas raide.

8. Ensemble de ponction selon l'une des revendications précédentes, **caractérisé en ce que** l'aiguille de ponction (4) est pourvue d'un filetage (12) qui est en engagement vissé avec un taraudage (11) ménagé dans le boîtier (1).

9. Ensemble de ponction selon l'une des revendications précédentes, **caractérisé en ce que** dans la zone de l'extrémité libre du talon tubulaire (3), des orifices transversaux (37, 37') sont ménagés dans la paroi du talon tubulaire.

10. Ensemble de ponction selon l'une des revendications précédentes, **caractérisé en ce qu'**il est prévu, sur le côté extérieur du talon tubulaire (3), un tronçon de paroi (23) radialement extensible sur sa périphérie, dont l'intérieur (24) est en liaison avec un raccord de pression (26) dans le tronçon de boîtier postérieur (2) via un canal sous pression (25).

11. Ensemble de ponction selon l'une des revendications précédentes, **caractérisé en ce que** l'extrémité postérieure de l'aiguille de ponction (4) débouche dans une chambre de boîtier (34) qui est en liaison avec le dispositif d'évacuation (14).

12. Ensemble de ponction selon l'une des revendications précédentes, **caractérisé en ce que** le dispositif d'évacuation (14) comprend une valve à trois voies dont l'entrée (16) est en liaison de fluide avec l'aiguille de ponction (4), dont la première sortie (17) est réalisée pour appliquer une seringue et dont la deuxième sortie (18) est en liaison de fluide avec un récipient de collecte.

13. Ensemble de ponction selon l'une des revendications précédentes, **caractérisé en ce qu'**une fenêtre d'observation (20 ; 35) est prévue dans le boîtier (1) ou dans la zone postérieure de l'aiguille de ponction (4).
